(19) Europäisches Patentamt
European Patent Office
Office européen des brevets

(11) Veröffentlichungsnummer: **0 023 307**
**A1**

(12) **EUROPÄISCHE PATENTANMELDUNG**

(21) Anmeldenummer: **80104063.5**

(22) Anmeldetag: **14.07.80**

(51) Int. Cl.³: **C 07 D 217/06**
A 01 N 25/32
//C07D217/04, C07D231/12,
C07D231/16, C07D249/08,
C07D249/10, C07D249/12,
C07D207/32, C07D257/04,
C07D233/61, C07D233/68,
C07D271/10, C07C103/42,
C07C109/097, C07C159/00

(30) Priorität: **26.07.79 DE 2930450**

(43) Veröffentlichungstag der Anmeldung:
**04.02.81 Patentblatt 81/5**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Riebel, Hans-Jochem, Dr.**
**In der Beek 92**
**D-5600 Wuppertal 1(DE)**

(72) Erfinder **Eue, Ludwig, Dr.**
**Paul-Klee-Strasse 36**
**D-5090 Leverkusen 1(DE)**

(72) Erfinder: **Faust, Wilfried, Dr.**
**Eifgenstrasse 16**
**D-5068 Odenthal 3(DE)**

(54) **N-(Alpha-Chlorpropionyl)-1,2,3,4-tetrahydro-iso-chinolin, Verfahren zu dessen Herstellung und dessen Verwendung als Gegenmittel zum Schutz von Kulturpflanzen vor Schädigungen durch Herbizide.**

(57) N-(α-Chlorpropionyl) -1,2,3,4-tetrahydro-isochinolin der Formel

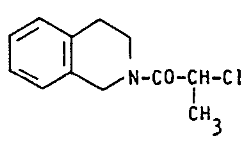

(1),

ein Verfahren zur Herstellung dieses Stoffes sowie dessen Verwendung als Gegenmittel zum Schutz con Kulturpflanzen vor Herbizidschäden.

Neue Wirkstoffkombinationen aus dem Stoff der Formel (I) und herbizid wirksamen Thiolcarbamaten bzw. Acetanilide.

EP 0 023 307 A1

BAYER AKTIENGESELLSCHAFT 5090 Leverkusen, Bayerwerk
Zentralbereich
Patente, Marken und Lizenzen Dü/kl
Ia

# N-(α-Chlorpropionyl)-1,2,3,4-tetrahydro-iso-chinolin, Verfahren zu dessen Herstellung und dessen Verwendung als Gegenmittel zum Schutz von Kulturpflanzen vor Schädigungen durch Herbizide

Die vorliegende Erfindung betrifft das neue N-(α-Chlor-propionyl)-1,2,3,4-tetrahydro-isochinolin, ein Verfahren zu dessen Herstellung sowie dessen Verwendung als Gegenmittel zum Schutz von Kulturpflanzen vor Schädigungen durch herbzid wirksame Thiolcarbamate und Acetanilide. Ferner betrifft die vorliegende Erfindung neue Wirkstoffkombinationen, die aus dem neuen N-(α-Chlor-propionyl)-1,2,3,4-tetrahydro-isochinolin und bestimmten herbizid wirksamen Thiolcarbamaten bzw. Acetaniliden bestehen und besonders gute selektive herbizide Eigenschaften besitzen.

Unter "Gegenmitteln" ("Safener", "Antidote") sind im vorliegenden Zusammenhang Stoffe zu verstehen, welche befähigt sind, schädigende Wirkungen von Herbiziden auf Kulturpflanzen spezifisch zu antagonisieren, d.h. die Kulturpflanzen zu schützen, ohne dabei die Herbizidwirkung auf die zu bekämpfenden Unkräuter merklich zu beeinflussen.



BAD ORIGINAL

Es ist bekannt, daß bestimmte Thiolcarbamate und Acetanilide beim Einsatz zur Unkrautbekämpfung in Mais und anderen Kulturen mehr oder weniger starke Schäden an den Kulturpflanzen hervorrufen. Weiterhin ist bekannt, daß Verbindungen wie z.B. N-Dichloracetyl-2-ethyl-piperidin und N-Dichloracetyl-cis,trans-decahydrochinolin geeignet sind, um Schädigungen durch Thiolcarbamate oder Acetanilide an Kulturpflanzen zu vermindern (vgl. DE-OS 22 18 097). Die Wirksamkeit dieser Stoffe als Gegenmittel ist jedoch nicht immer ganz befriedigend.

Es wurde jetzt das neue N-(α-Chlorpropionyl)-1,2,3,4-tetrahydro-isochinolin der Formel

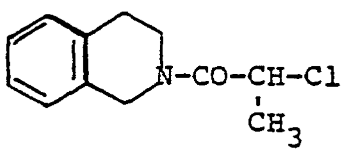

(I)

gefunden.

Weiterhin wurde gefunden, daß man das neue N-(α-Chlorpropionyl)-1,2,3,4-tetrahydro-isochinolin der Formel (I) erhält, wenn man 1,2,3,4-Tetrahydro-isochinolin der Formel

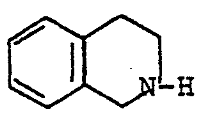

(II)

mit α-Chlor-propionsäurechlorid der Formel

$$\underset{\displaystyle Cl}{\text{Cl-CO-CH-CH}_3} \qquad (III)$$

gegebenenfalls in Gegenwart eines Säurebindemittels sowie gegebenenfalls in Gegenwart eines Verdünnungsmittels umsetzt.

Ferner wurde gefunden, daß N-( $\alpha$ -Chlorpropionyl)-1,2,3,4-tetrahydro-isochinolin der Formel (I) hervorragend geeignet ist, um Kulturpflanzen vor Schädigungen durch herbizid wirksame Thiolcarbamate bzw. durch herbizid wirksame Acetanilide zu schützen.

Außerdem wurde gefunden, daß die neuen Wirkstoffkombinationen bestehend aus dem N-($\alpha$ -Chlorpropionyl)-1,2,3,4-tetrahydro-isochinolin der Formel (I) und mindestens einem herbizid wirksamen Thiolcarbamat bzw. mindestens einem herbizid wirksamen Acetanilid hervorragend geeignet sind zur selektiven Unkrautbekämpfung in Nutzpflanzenkulturen.

Überraschenderweise werden Herbizidschädigungen durch Thiolcarbamate bzw. durch Acetanilide an Kulturpflanzen bei Mitverwendung von N-( $\alpha$ -Chlorpropionyl)-1,2,3,4-tetrahydro-isochinolin der Formel (I) besser unterdrückt als beim Einsatz der bekannten Verbindungen N-Dichlor-acetyl-cis,trans-decahydrochinolin und N-Dichloracetyl-2-ethyl-piperidin, welches chemisch ähnliche Stoffe gleicher Wirkungsart sind. Im übrigen war nicht zu erwarten, daß die erfindungsgemäßen

Wirkstoffkombinationen bessere selektive herbizide Eigenschaften besitzen als Wirkstoffkombinationen, welche aus mindestens einem herbizid wirksamen Thiolcarbamat bzw. mindestens einem herbizid wirksamen Acetanilid und dem als Gegenmittel bekannten N-Dichloracetyl-2-ethyl-piperidin oder dem ebenfalls als Gegenmittel bekannten N-Dichloracetyl-cis,trans-decahydrochinolin bestehen. Der erfindungsgemäße Stoff stellt somit eine wertvolle Bereicherung der Technik dar.

Die Herstellung des N-(α-Chlorpropionyl)-1,2,3,4-tetrahydro-isochinolins durch Umsetzung von 1,2,3,4-Tetrahydro-isochinolin mit α-Chlor-propionsäurechlorid läßt sich durch das folgende Formelschema veranschaulichen:

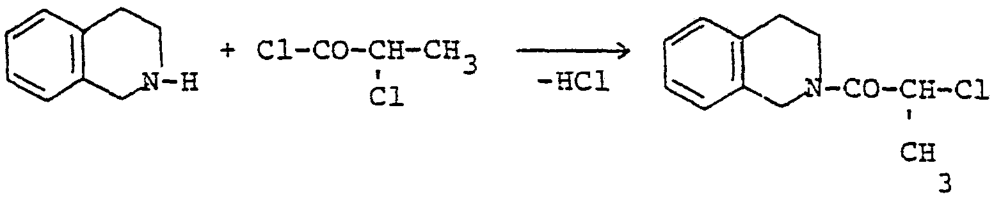


Das bei der Durchführung des erfindungsgemäßen Verfahrens als Ausgangsprodukt benötigte 1,2,3,4-Tetrahydro-isochinolin läßt sich durch Hydrierung von Isochinolin mit gasförmigem Wasserstoff in Gegenwart eines Katalysators, wie Raney-Nickel oder Ruthenium auf einem Trägerstoff, sowie gegebenenfalls in Gegenwart eines Verdünnungsmittels, wie Methanol oder Ethanol, bei Temepraturen zwischen 100°C und

250 °C, vorzugsweise zwischen 150 °C und 200 °C herstellen. Der Wasserstoffdruck kann innerhalb eines größeren Bereiches variiert werden. Im allgemeinen arbeitet man bei Drucken zwischen 100 und 200 bar, vorzugsweise zwischen 150 und 190 bar. Bevorzugt wird die Hydrierung von Isochinolin in Abwesenheit zusätzlicher Verdünnungsmittel unter Verwendung von Ruthenium auf Aluminiumoxid als Katalysator vorgenommen.

Das α-Chlor-propionsäurechlorid, das bei dem erfindungsgemäßen Verfahren als Reaktionskomponente benötigt wird, ist bekannt.

Bei der Durchführung des erfindungsgemäßen Verfahrens kommen als Säurebindemittel alle üblichen Säureakzeptoren in Betracht. Hierzu gehören vorzugsweise Alkalihydroxide, wie Natriumhydroxid und Kaliumhydroxid, ferner Alkalicarbonate, wie Natriumcarbonat, Kaliumcarbonat und Natriumhydrogencarbonat, und weiterhin niedere tertiäre Amine, wie Triäthylamin, Dimethylbenzylamin, Pyridin und Diazabicyclooctan. Es kann jedoch auch im Überschuß eingesetztes 1,2,3,4-Tetrahydro-isochinolin der Formel (II) gleichzeitig als Säurebindemittel fungieren. In diesem Fall erübrigt sich die Zugabe eines zusätzlichen Säurebindemittels.

Als Verdünnungsmittel können bei der erfindungsgemäßen Umsetzung Wasser sowie inerte organische Lösungsmittel verwendet werden. Hierzu gehören vorzugsweise Ketone, wie Diäthylketon und Methylisobutylketon; Nitrile,



BAD ORIGINAL

wie Propionitril und Acetonitril; Aether, wie Tetrahydrofuran oder Dioxan; aliphatische und aromatische Kohlenwasserstoffe, wie Petroläther, Benzol, Toluol oder Xylol, halogenierte Kohlenwasserstoffe, wie Methylenchlorid, Tetrachlorkohlenstoff, Chloroform oder Chlorbenzol; Ester, wie Essigester; und Formamide, wie insbesondere Dimethylformamid.

Die Reaktionstemperaturen können beim erfindungsgemäßen Verfahren in einem größeren Bereich variiert werden. Im allgemeinen arbeitet man zwischen etwa 0 und 60°C, vorzugsweise zwischen 20 und 50°C.

Bei der Durchführung des erfindungsgemäßen Verfahrens setzt man auf 1 Mol 1,2,3,4-Tetrahydro-isochinolin der Formel (II) vorzugsweise 1 Mol $\alpha$-Chlor-propionsäurechlorid der Formel (III) und 1 Mol Säurebindemittel ein. Die Isolierung des Reaktionsproduktes erfolgt nach üblichen Methoden. Im allgemeinen verfährt man in der Weise, daß man das Reaktionsgemisch nach beendeter Umsetzung nacheinander mit Wasser und einem in Wasser wenig löslichen organischen Solvens, wie z.B. Methylenchlorid oder Toluol, versetzt, die organische Phase abtrennt, wäscht, dann trocknet und eindampft und den verbleibenden Rückstand andestilliert.

Das erfindungsgemäße N-($\alpha$-Chlorpropionyl)-1,2,3,4-tetrahydro-isochinolin der Formel (I) eignet sich, - wie bereits erwähnt - , zum Schutz von Kulturpflanzen vor Schädigungen durch herbizid wirksame Thiolcarbamate und Acetanilide, ohne deren Unkrautwirkung merklich zu beeinflussen.

Vorzugsweise kann das N-( α-Chlorpropionyl)-1,2,3,4-tetrahydro-isochinolin der Formel (I) als Gegenmittel zum Schutz von Kulturpflanzen vor Schädigungen durch herbizid wirksame Thiolcarbamate der Formel

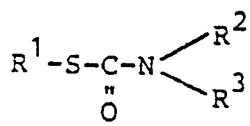

(IV)

verwendet werden,

in welcher

$R^1$ für niederes Alkyl, Benzyl, Chlorbenzyl oder Alkoxybenzyl steht,

$R^2$ und $R^3$ unabhängig voneinander für Alkyl mit 2 bis 4 Kohlenstoffatomen oder für Cyclohexyl stehen und außerdem

$R^2$ und $R^3$ gemeinsam mit dem angrenzenden Stickstoffatom für einen fünf- bis siebengliedrigen heterocyclischen Ring stehen,

bzw. zum Schutz von Kulturpflanzen vor Schädigungen durch herbizid wirksame Acetanilide

- der Formel

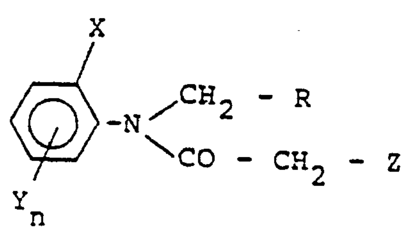

(V)

in welcher

R für einen gegebenenfalls substituierten N-haltigen heterocyclischen Rest steht,

X und Y gleich oder verschieden sind und für Alkyl stehen,

Z für Halogen steht und

n für 0, 1 oder 2 steht,

sowie deren herbizid-wirksame Säureadditionssalze und Metallsalz-Komplexe,

- beziehungsweise der Formel

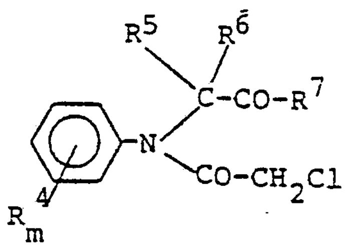

(VI)

in welcher

$R^4$ für Alkyl, Halogen, Halogenalkyl, Alkylthio, Alkylsulfonyl, Aminosulfonyl, Cyano oder Nitro steht,

$R^5$ und $R^6$ gleich oder verschieden sind und für Wasserstoff, Alkyl, Halogen, Halogenalkyl oder gegebenenfalls substituiertes Phenyl stehen,

$R^7$ für Alkyl oder gegebenenfalls substituiertes Phenyl steht und

m für ganze Zahlen von 0 bis 5 steht,

oder der Formel

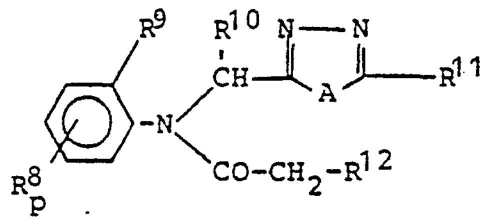

(VII)

in welcher

A für Sauerstoff, Schwefel oder die Gruppierung $NR^{13}$ steht,

$R^{10}$ für Wasserstoff oder Alkyl steht,

$R^{11}$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^{14}$, $-SR^{14}$ und $NR^{13}R^{14}$ steht,

$R^{13}$ für Wasserstoff, Alkyl oder gegebenenfalls substituiertes Aryl steht,

$R^{14}$ für Wasserstoff, Alkyl, Halogenalkyl, Alkenyl, Alkinyl, Cycloalkyl oder gegebenenfalls substituiertes Aralkyl steht,

$R^8$ für Alkyl steht,

$R^9$ für Alkyl oder Halogen steht,

$R^{12}$ für Halogen steht und

p für die Zahlen 0, 1 oder 2 steht,

- beziehungsweise der Formel

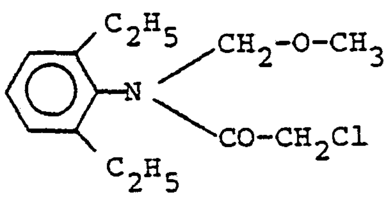

(VIII)

- oder der Formel

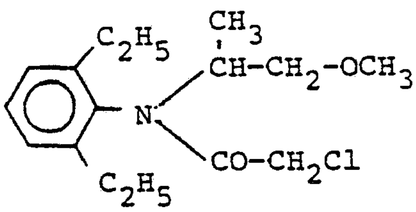

(IX)

verwendet werden.

Als Beispiele für Thiolcarbamate der Formel (IV) seien im einzelnen genannt:

S-Ethyl-N,N-dipropylthiocarbamat
S-Ethyl-N,N-diisobutylthiocarbamat
S-Propyl-N-butyl-N-ethylthiocarbamat

S-Propyl-N,N-diisopropylthiocarbamat
S-Ethyl-N,N-diethylthiocarbamat
S-Ethyl-N-ethyl-N-cyclohexylthiocarbamat
S-Ethyl-hexahydro-azepin-1-thiocarbamat
S-p-Methoxybenzyl-N,N-diethylthiocarbamat
S-p-Chlorbenzyl-N,N-diethylthiocarbamat
S-Benzyl-N,N-diethylthiocarbamat
S-Benzyl-N,N-di-sek.-butylthiocarbamat
S-Propyl-N-ethyl-N-butylthiocarbamat

Die Thiolcarbamate der Formel (IV) und deren herbizide Wirksamkeit sind bereits bekannt (vgl. US-Patentschriften 2 913 327, 3 o37 853, 3 175 897, 3 185 72O, 3 198 786 und 3 582 314).

In der Formel (V) steht R vorzugsweise für die gegebenenfalls substituierten Azolylreste Pyrazol-1-yl, Imidazol-1-yl, 1,2,4-Triazol-1-yl; 1,2,3-Triazol-1-yl; 1,3,4-Triazol-1-yl und 1,2,3,4-Tetrazol-1-yl sowie für gegebenenfalls substituiertes Pyrrol-1-yl. Als Substituenten kommen vorzugsweise in Frage: Halogen, insbesondere Fluor, Chlor und Brom, sowie Alkyl mit 1 bis 4 Kohlenstoffatomen. X und Y sind gleich oder verschieden und stehen vorzugsweise für geradkettiges oder verzweigtes Alkyl mit 1 bis 4 Kohlenstoffatomen. Z steht vorzugsweise für die Halogene Chlor und Brom und der Index m steht für O, 1 oder 2.

Als Beispiele für Acetanilide der Formel (V) seien im einzelnen genannt:

2-Methyl-6-ethyl-N-(pyrazol-1-yl-methyl)-chloracetanilid

2,6-Diethyl-N-(pyrazol-1-yl-methyl)-chloracetanilid

2,6-Diethyl-N-(1,2,4-triazol-1-yl-methyl)-chloracetanilid

2,6-Dimethyl-N-(1,2,4-triazol-1-yl-methyl)-chloracetanilid

2-Methyl-N-(pyrazol-1-yl-methyl)-chloracetanilid

2,5-Dimethyl-N-(pyrazol-1-yl-methyl)-chloracetanilid

2,3-Dimethyl-N-(pyrazol-1-yl-methyl)-chloracetanilid

2-Methyl-6-ethyl-N-(pyrazol-1-yl-methyl)-chloracetanilid-hydrochlorid

2,6-Diethyl-N-(pyrazol-1-yl-methyl)-chloracetanilid-hydrochlorid

2,6-Diethyl-N-[(3,5-dimethyl-pyrazol-1-yl)-methyl]-chloracetanilid

2,6-Diethyl-N-[(3-chlor-1,2,4-triazolyl)-methyl]-chloracetanilid

2-Methyl-6-ethyl-N-[(3,5-dimethyl-pyrazol-1-yl)-methyl]-chloracetanilid

2-tert.-Butyl-N-(pyrazol-1-yl-methyl)-chloracetanilid

2-Methyl-6-ethyl-N-[(3-brom-5-methyl-pyrazol-1-yl)-methyl]-chloracetanilid

2-Methyl-6-ethyl-N-[(3-chlor-1,2,4-triazolyl)-methyl]-chloracetanilid

2,6-Diethyl-N-[(4-chlor-pyrazol-1-yl)-methyl]-chloracetanilid

Weitere bevorzugt in Frage kommende Acetanilide der Formel (V) sind in den Herstellungsbeispielen aufgeführt.

Die Acetanilide der Formel (V) und deren herbizide Wirksamkeit sowie deren herbizid wirksame Säureadditionssalze und Metallsalz-Komplexe sind bereits bekannt (vgl. DE-OS 26 48 008 und DE-OS 27 04 281).

In der Formel (VI) steht $R^4$ vorzugsweise für geradkettiges oder verzweigtes Alkyl mit 1 bis 6, insbesondere 1 bis 4 Kohlenstoffatomen, Halogen, insbesondere Fluor, Chlor oder Brom, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt; ferner vorzugsweise für Alkylthio und Alkylsulfonyl mit 1 bis 4 Kohlenstoffatomen im Alkylteil sowie für Aminosulfonyl, Cyano und Nitro. $R^5$ und $R^6$ sind gleich oder verschieden und stehen vorzugsweise für Wasserstoff, geradkettiges oder verzweigtes Alkyl mit 1 bis 4 Kohlenstoffatomen, Halogen, insbesondere Fluor, Chlor und Brom, Halogenalkyl mit bis zu 3 Kohlenstoff- und bis zu 5 gleichen oder verschiedenen Halogenatomen, wobei als Halogene insbesondere Fluor und Chlor stehen sowie vorzugsweise für gegebenenfalls einfach oder mehrfach substituiertes Phenyl, wobei als Substituenten vorzugsweise die für $R^4$ genannten Reste in Frage

kommen. $R^7$ steht vorzugsweise für geradkettiges oder verzweigtes Alkyl mit 1 bis 6, insbesondere 1 bis 4 Kohlenstoffatomen sowie für gegebenenfalls einfach oder mehrfach substituiertes Phenyl, wobei als Substituenten vorzugsweise in Frage kommen: Alkyl mit 1 bis 4 Kohlenstoffatomen, Halogen, insbesondere Fluor, Chlor und Brom, Halogenalkyl mit bis zu 3 Kohlenstoffatomen und bis zu 5 gleichen oder verschiedenen Halogenatomen, insbesondere Fluor- oder Chloratome, wobei Trifluormethyl beispielhaft genannt sei, ferner Alkoxy, Alkylthio und Alkylsulfonyl mit jeweils 1 bis 4 Kohlenstoffatomen, weiterhin Aminosulfonyl, Cyano und Nitro sowie gegebenenfalls durch Chlor substituiertes Phenyl oder Phenoxy. Der Index m steht vorzugsweise für 1, 2 oder 3.

Als Beispiele für Acetanilide der Formel (VI) seien im einzelnen genannt:
2,6-Dimethyl-N-(benzoyl-methyl)-chloracetanilid
2,6-Dimethyl-N-(4-chlorbenzoyl-methyl)-chloracetanilid
2-Methyl-6-ethyl-N-(benzoyl-methyl)-chloracetanilid

Weitere bevorzugt in Frage kommende Acetanilide der Formel (VI) sind in den Herstellungsbeispielen aufgeführt.

Die Acetanilide der Formel (VI) und deren herbizide Wirksamkeit sind bereits bekannt (vgl. DE-OS 27 26 253).

In der Formel (VII) steht A vorzugsweise für Sauerstoff, Schwefel oder die Gruppierung $-NR^{13}$, worin $R^{13}$ vorzugsweise für Wasserstoff, geradkettiges oder verzweigtes Alkyl mit 1 bis 4 Kohlenstoffatomen sowie für Aryl mit 6 bis 10 Kohlenstoffatomen, insbesondere Phenyl, steht, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbesondere Fluor und Chlor genannt seien. $R^{10}$ steht vorzugsweise für Wasserstoff oder Methyl. $R^{11}$ steht in der Formel (VII) vorzugsweise für Wasserstoff, geradkettiges oder verzweigtes Alkyl mit 1 bis 4 Kohlenstoffatomen, Halogenalkyl mit bis zu 3 Kohlenstoffatomen und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Halogen, insbesondere Fluor, Chlor oder Brom. $R^{11}$ steht ferner vorzugsweise für Aryl mit 6 bis 10 Kohlenstoffatomen, insbesondere Phenyl, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^{11}$ steht ferner vorzugsweise für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Außerdem steht $R^{11}$ für die Gruppierung $-OR^{14}$, $-SR^{14}$ und $-NR^{13}R^{14}$, worin $R^{13}$ vorzugsweise für diejenigen Reste steht, die oben bereits vorzugsweise für diesen Rest genannt wurden, $R^{14}$ steht in diesen Gruppierungen für Wasserstoff, geradkettiges oder verzweigtes Alkyl mit 1 bis 4 Kohlenstoffatomen, Halogenalkyl mit 1 bis 3 Kohlenstoffatomen und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. In der Formel (VII) steht $R^8$ vorzugsweise für geradkettiges oder verzweigtes Alkyl mit 1 bis 4 Kohlenstoffatomen. $R^9$ steht in der Formel (VII) vorzugsqeise für geradkettiges oder verzweigtes Alkyl mit 1 bis 4 Kohlenstoffatomen oder für die Halogene Fluor, Chlor und Brom. $R^{12}$ steht in der Formel (VII) vorzugsweise für Chlor, Brom und Jod. Der Index p steht für die Zahlen 0, 1 oder 2.

Als Beispiele für Acetanilide der Formel (VII) seien im einzelnen genannt:

2,6-Diethyl-N-[(2-methyl-1,3,4-oxadiazol-5-yl)-methyl]-chloracetanilid

2,6-Dimethyl-N-[(2-methyl-1,3,4-oxadiazol-5-yl)-methyl]-chloracetanilid
2-Ethyl-6-methyl-N-[(2-methyl-1,3,4-oxadiazol-5-yl)-methyl]-chloracetanilid
2-tert.-Butyl-N-[(2-methyl-1,3,4-oxadiazol-5-yl)-methyl]-chloracetanilid

Weitere bevorzugt in Frage kommende Acetanilide der Formel (VII) sind in den Herstellungsbeispielen aufgeführt.

Die Acetanilide der Formel (VII) und deren herbizide Wirksamkeit sind bisher noch nicht bekannt. Sie lassen sich in einfacher Weise herstellen. So erhält man Acetanilide der Formel (VII), indem man N-Azolyl-alkylaniline der Formel

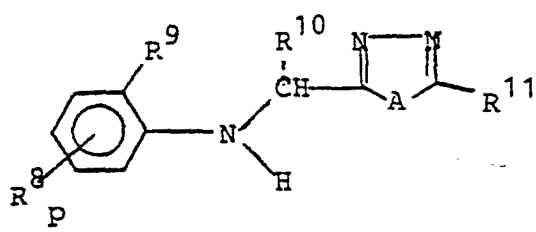

(X)

in welcher

$R^8$, $R^9$, $R^{10}$, $R^{11}$, A und p die oben angegebene Bedeutung haben,

mit Halogenessigsäurechloriden bzw. -anhydriden der Formeln

$R^{12}-CH_2-CO-Cl$ (XIa)

bzw.

$(R^{12}-CH_2-CO)_2O$ (XIb)

in welchen

$R^{12}$ die oben angegebene Bedeutung hat,

in Gegenwart eines Verdünnungsmittels und gegebenenfalls in Gegenwart eines Säurebindemittels umsetzt.

Verwendet man 2,6-Diethyl-N-(3-methylthio-4-methyl-1,2,4-triazol-5-yl-methyl)-anilin und Chloracetylchlorid als Ausgangsstoffe, so kann der Reaktionsablauf des Verfahrens zur Herstellung der Acetanilide der Formel (VII) durch das folgende Formelschema wiedergegeben werden:

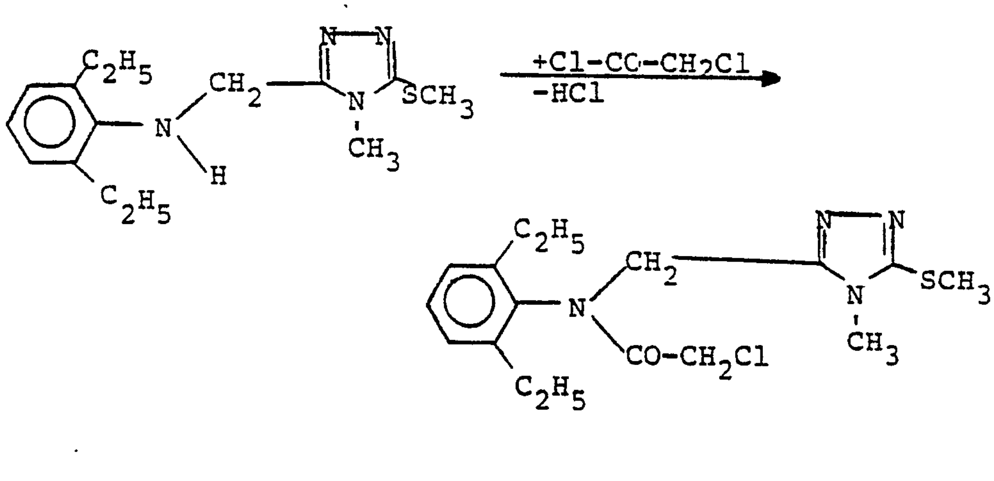

Die bei der Durchführung des Verfahrens zur Herstellung der Acetanilide der Formel (VII) als Ausgangsstoffe benötigten N-Azolylalkylaniline sind durch die Formel (X) allgemein definiert. In dieser Formel stehen $R^8$, $R^9$, $R^{10}$, $R^{11}$, A und p vorzugsweise für diejenigen Reste, die bereits im Zusammenhang mit der Beschreibung der Acetanilide der Formel (VII) vorzugsweise für diese Reste bzw. für den Index p genannt wurden.

Die bei dem Verfahren zur Herstellung der Acetanilide der Formel (VII) als Ausgangsstoffe benötigten N-Azolylalkylaniline der Formel (X) sind noch nicht bekannt. Sie lassen sich jedoch in einfacher Weise nach mehreren Verfahren herstellen. So erhält man N-Azolylalkylaniline der Formel (X), indem man

a) Aniline der Formel

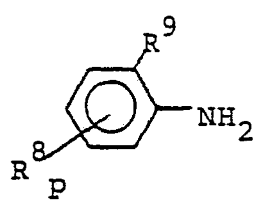

(XII)

in welcher

$R^8$, $R^9$ und p die oben angegebene Bedeutung haben,

mit Azolderivaten der Formel

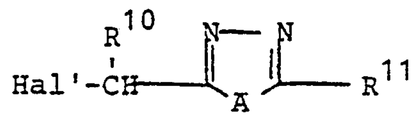

(XIII)

in welcher

A, $R^{10}$ und $R^{11}$ die oben angegebene Bedeutung haben und

Hal' für Chlor oder Brom steht,

in Gegenwart eines Säurebinders, wie beispielsweise Kalium- oder Natriumcarbonat, und in Gegenwart eines inerten organischen Lösungsmittels, wie beispielsweise Dimethylformamid oder Toluol, bei Temperaturen zwischen 20 und 160°C umsetzt, wobei vorzugsweise ein Überschuß an Anilin der Formel (XII) eingesetzt wird (vgl. auch Herstellungsbeispiele), oder

b) Hydrazin-Derivate der Formel

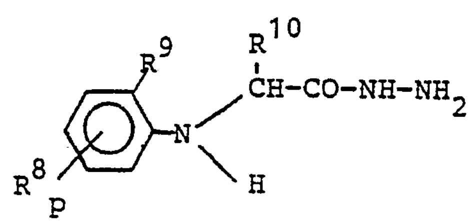

(XIV)

in welcher

$R^8$, $R^9$, $R^{10}$ und p die oben angegebene Bedeutung haben,

mit Isocyanaten bzw. Senfölen der Formel

$$R^{13}-N=C=B \qquad (XV)$$

in welcher

B für Sauerstoff oder Schwefel steht und

$R^{13}$ die oben angegebene Bedeutung hat,

in Gegenwart eines organischen Lösungsmittels, wie beispielsweise eines Alkohols, Ethers oder Kohlenwasserstoffs, bei Temperaturen zwischen 0 und 80°C umsetzt, die entstehenden Verbindungen der Formel

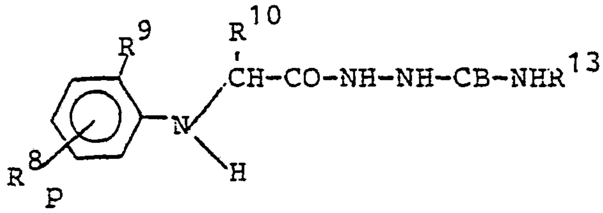

(XVI)

in welcher

B, $R^8$, $R^9$, $R^{10}$, $R^{13}$ und p die oben angegebene Bedeutung haben,

in Gegenwart einer starken Base, wie beispielsweise Natron- oder Kalilauge, und in Gegenwart eines Lösungsmittels, wie beispielsweise Ethanol oder Wasser bei Temperaturen zwischen 20 und

100°C cyclisiert und die entstehenden Triazolone bzw. Triazolthione der Formel

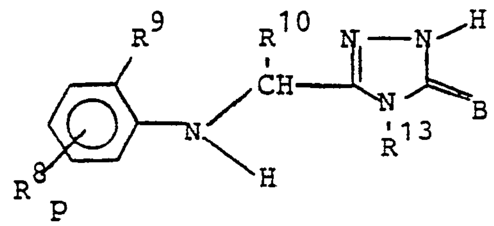

(XVII)

in welcher

B, $R^8$, $R^9$, $R^{10}$, $R^{13}$ und p die oben angegebene Bedeutung haben,

mit Halogeniden der Formel

Hal'-$R^{15}$ (XVIII)

in welcher

Hal' für Chlor oder Brom steht und

$R^{15}$ für die Reste des Substituenten $R^{14}$ steht, wobei Wasserstoff ausgenommen ist,

in Gegenwart einer starken Base, wie beispielsweise Natronlauge, und in Gegenwart eines inerten organischen Lösungsmittels, wie beispielsweise Toluol oder Methylenchlorid, bei Temperaturen zwischen 20 und 80°C umsetzt, wobei auch phasentransferkatalysiert und mit anderen Alkylierungsreagenzien, wie beispielsweise Dimethylsulfat, gearbeitet werden kann (vgl. auch Herstellungsbeispiele), oder

c) Hydrazin-Derivate der Formel (XIV) mit Ameisensäure oder Säurechloriden bzw. Säureanhydriden der Formeln

$$R^{16}-CO-Cl \qquad (XIXa)$$

bzw.

$$(R^{16}-CO-)_2O \qquad (XIXb)$$

in welchen

$R^{16}$ für Alkyl, Halogenalkyl, Alkenyl, Alkinyl, Cycloalkyl, gegebenenfalls substituiertes Aryl oder gegebenenfalls substituiertes Aralkyl steht

in Gegenwart eines inerten organischen Lösungsmittels, wie eines Ethers, Kohlenwasserstoffs oder Halogenkohlenwasserstoffs, bei Temperaturen zwischen 0 und 50°C umsetzt und die entstehenden Verbindungen der Formel

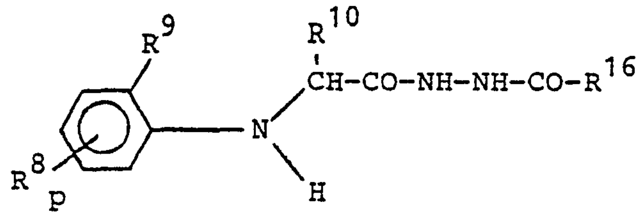

(XX)

in welcher

$R^8$, $R^9$, $R^{10}$, $R^{16}$ und p die oben angegebene Bedeutung haben,

entweder mit Diphosphorpentasulfid in an sich bekannter Weise (vgl. Chem. Ber. 32, 797 (1899) und J.prakt. Chemie 69, 145 (1904)) zu Thiadiazol-Derivaten cyclisisert, oder ebenfalls in bekannter Weise mit üblichen wasserabspaltenden Reagenzien zu Oxadiazol-Derivaten umsetzt (vgl. hierzu Elderfield, Heterocyclic Compounds, Vol. 7 (1961)) oder

d) Hydrazin-Derivate der Formel (XIV) mit Nitrilen der Formel

$$R^{17}-C\equiv N \qquad (XXI)$$

in welcher

$R^{17}$ für Alkyl, Halogenalkyl oder gegebenenfalls substituiertes Aryl steht,

in an sich bekannter Weise zu Triazol-Derivaten umsetzt (vgl. Chem.Ber. 96, 1064 (1963)), oder

e) Hydrazin-Derivate der Formel (XIV) mit Iminoethern der Formel

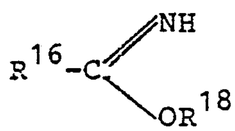 x HCl (XXII)

in welcher

$R^{16}$ für Alkyl, Halogenalkyl, Alkenyl, Alkinyl, Cycloalkyl, gegebenenfalls substituiertes Aryl oder gegebenenfalls substituiertes Aralkyl steht und

$R^{18}$ für Methyl oder Ethyl steht,

in an sich bekannter Weise unter Rückfluß und in Gegenwart eines inerten organischen Lösungsmittels, wie beispielsweise Ethanol, zu Oxadiazol-Derivaten umsetzt, oder

f) die Aniline der Formel (XII) mit Azol-aldehyden der Formel

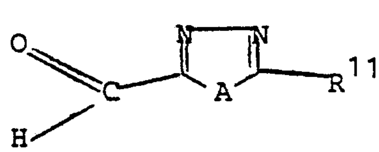 (XXIII)

in welcher

$R^{11}$ die oben angegebene Bedeutung hat,

in Gegenwart eines inerten organischen Lösungsmittels, wie beispielsweise Toluol, bei Temperaturen zwischen 80 und 120°C umsetzt und die entstehenden Verbindungen der Formel

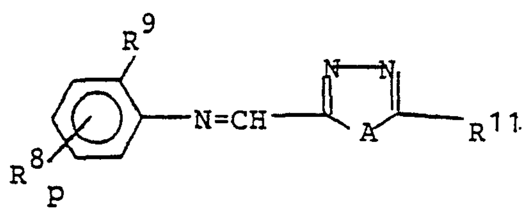

(XXIV)

in welcher

A, $R^8$, $R^9$, $R^{11}$ und p die oben angegebene Bedeutung haben,

in allgemein bekannter Weise reduziert; z.B. durch Umsetzung mit komplexen Hydriden, wie Natriumborhydrid, gegebenenfalls in Gegenwart eines polaren organischen Lösungsmittels, wie Methanol, bei Temperaturen zwischen 0 und 80°C.

Die bei dem Verfahren a) als Ausgangsstoffe benötigten Verbindungen der Formeln (XII) und (XIII) sind bekannt oder lassen sich nach im Prinzip bekannten Verfahren herstellen (vgl. Helv. Chim. Acta 55, 19 9 ff (1972), Chem. Ber. 32, 797 ff (1899) und

Chem. Ber. 96, 1049 ff (1963)).

Die bei dem Verfahren b) benötigten Ausgangsstoffe der Formel (XIV) sind noch nicht bekannt. Sie lassen sich jedoch nach bekannten Verfahren herstellen, indem man bekannte Ester (vgl. u.a. DE-OS 2 350 944 und 2 513 730) der Formel

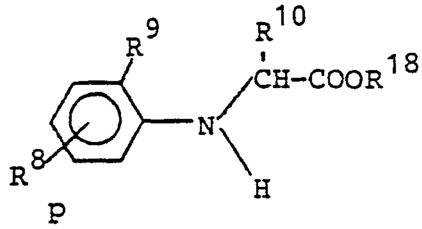

(XXV)

in welcher

$R^8$, $R^9$, $R^{10}$ und p die oben angegebene Bedeutung haben und

$R^{18}$ für Methyl oder Ethyl steht,

mit Hydrazinhydrat vorzugsweise in Gegenwart eines organischen Lösungsmittels, wie beispielsweise Ethanol, Dioxan oder Dimethylformamid, bei Temperaturen zwischen 20 und 120°C umsetzt (vgl. auch Herstellungsbeispiele).

Die bei dem Verfahren b) benötigten Reaktionskomponenten der Formeln (XV) und (XVIII) sind allgemein bekannte Verbindungen der organischen Chemie.

Die bei den Verfahren c), d) und e) als Reaktionskomponenten benötigten Stoffe der Formel (XIXa), (XIXb), (XXI) und (XXII) sind ebenfalls bekannt.

Die bei dem Verfahren f) als Reaktionskomponenten zu verwendenden Azol-aldehyde der Formel (XXIII) sind ebenfalls bekannt oder lassen sich nach im Prinzip bekannten Verfahren herstellen (vgl. Elderfield, "Heterocyclic Compounds", Vol. 7 (1961) und "Advances in Heterocyclic" Chemistry, Vol. 9 (1968)).

Die außerdem bei der Herstellung der Acetanilide der Formel (VII) als Ausgangsstoffe benötigten Halogenessigsäurechloride bzw. -anhydride sind durch die Formeln (XIa) und (XIb) allgemein definiert. In diesen Formeln steht $R^{12}$ vorzugsweise für Chlor, Brom und Jod.

Die Halogenessigsäurechloride und -anhydride der Formeln (XIa) und (XIb) sind allgemein bekannten Verbindungen der organischen Chemie.

Als Verdünnungsmittel kommen für die Umsetzung zur Herstellung der Acetanilide der Formel (VII) vorzugsweise inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Ether wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das Verfahren zur Herstellung von Acetanilinen der Formel (VII) kann gegebenenfalls in Gegenwart von Säurebindern (Chlorwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder wie Pyridin; ferner anorganische Basen, wie beispielsweise Alkalihydroxide und Alkalicarbonate.

Die Reaktionstemperaturen können bei der Durchführung des Verfahrens zur Herstellung der Acetanilide der Formel (VII) in einem größeren Bereich variiert werden. Im allgemeinen arbeitet man bei Temperaturen zwischen 0°C und 120°C, vorzugsweise zwischen 20°C und 100°C.

Bei der Durchführung des Verfahrens zur Herstellung der Acetanilide der Formel (VII) setzt man vorzugsweise auf 1 Mol der Verbindung der Formel (X) 1 bis 1,5 Mol Halogenacetylierungsmittel und 1 bis 1,5 Mol Säurebinder ein. Die Isolierung der Verbindungen der Formel (VII) erfolgt in üblicher Weise.

Weitere bevorzugt in Frage kommende Acetanilide, bei denen die erfindungsgemäße Verbindung der Formel (I)

als Gegenmittel eingesetzt werden kann, sind die Verbindungen der Formeln (VIII) und (IX). Diese Stoffe und deren herbizide Wirksamkeit sind bereits bekannt (vgl. US-PS 3 442 945 und DE-OS 23 28 340).

Das erfindungsgemäße N-( $\chi$ -Chlor-propionyl)-1,2,3,4-tetrahydro-isochinolin der Formel (I) eignet sich insbesondere zum Schutz von wichtigen Kulturpflanzen, wie Mais, Sojabohnen, Baumwolle, Zuckerrüben, Getreide, Reis und Zuckerrohr vor Herbizidschädigungen durch Thiolcarbamate und Acetanilide.

Die erfindungsgemäßen Wirkstoffkombinationen bestehend aus dem N-( $\chi$ -Chlorpropionyl)-1,2,3,4-tetrahydro-isochinolin der Formel (I) und mindestens einem herbizid wirksamen Thiolcarbamat bzw. mindestens einem herbizid wirksamen Acetanilid zeigen eine sehr gute Wirkung gegen Unkräuter und Ungräser in zahlreichen Nutzpflanzenkulturen. Sie können daher zu selektiven Unkrautbekämpfung in zahlreichen Nutzpflanzenkulturen verwendet werden.
- Unter Unkräutern im weitesten Sinne sind hierbei alle Pflanzen zu verstehen, die an Orten wachsen, wo sie unerwünscht sind.

Die erfindungsgemäßen Wirkstoffkombinationnen können z.B. bei folgenden Pflanzen angewendet werden:

Dikotyle Unkräuter der Gattungen: Sinapis, Lepidium, Galium, Stellaria, Matricaria, Anthemis, Galinsoga,

Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum Linum, Ipomoea, Vicia, Nicotiana Lycopersicon, Arachis, Brassica, Lactuca; Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Insbesondere eignen sich die erfindungsgemäßen Wirkstoffkombinationen zur selektiven Unkrautbekämpfung in Mais, Sojabohnen, Baumwolle, Zuckerrüben, Getreide, Reis und Zuckerrohr.

Das erfindungsgemäße Gegenmittel kann gegebenenfalls im Gemisch mit den herbiziden Wirkstoffen, bei denen es eingesetzt wird, in die üblichen Formulierungen

übergeführt werden, wie Lösungen, Emulsionen, Spritzpulver, Suspensionen, Pulver, Stäubemittel, Schäume, Pasten, lösliche Pulver, Granulate, Suspensions-Emulsionskonzentrate, wirkstoffimprägnierte Natur- und synthetische Stoffe, Feinstverkapselungen in polymeren Stoffen und in Hüllmassen für Saatgut.

Diese Formulierungen werden in bekannter Weise hergestellt, z.B. durch Vermischen des erfindungsgemäßen Gegemittels gegebenenfall im Gemisch mit den herbiziden Wirkstoffen, bei denen es eingesetzt wird, mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; als feste Trägerstoffe:

natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine, wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgier- und/oder schaumerzeugende Mittel: nichtionogene und anionische Emulgatoren, wie Polyoxiethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% an Gegenmittel bzw. an Gegenmittel und herbizidem Wirkstoff, vorzugsweise zwischen 0,5 und 90 %.

Das erfindungsgemäße Gegenmittel kann als solches oder in seinen Formulierungen auch in Mischung mit herbiziden Wirkstoffen eingesetzt werden, wobei Fertigformulierung oder Tankmischung möglich ist. Auch eine Mischung mit anderen bekannten Wirkstoffen wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Das erfindungsgemäße Gegenmittel bzw. Gemische aus dem erfindungsgemäßen Gegenmittel und herbizidem Wirkstoff können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Gießen, Spritzen, Sprühen, Stäuben, Streuen, Trockenbeizen, Feuchtbeizen, Naßbeizen, Schlämmbeizen oder Inkrustieren.

Das erfindungsgemäße Gegenmittel kann nach den für derartige Antidote üblichen Methoden ausgebracht werden. So kann das erfindungsgemäße Gegenmittel

entweder vor oder nach dem Herbizid ausgebracht oder zusammen mit dem Herbizid appliziert werden. Ferner können Kulturpflanzen durch Saatgutbehandlung mit dem Gegenmittel vor der Saat (Beizung) vor Schäden geschützt werden, wenn das Herbizid vor oder nach der Saat angewendet wird. Eine weitere Einsatzmöglichkeit besteht darin, daß man das Gegenmittel bei der Aussaat in die Saatfurche ausbringt. Wenn es sich bei den Pflanzen um Stecklinge handelt, so können diese vor der Auspflanzung mit dem Gegenmittel behandelt werden.

Beim Einsatz des erfindungsgemäßen Gegenmittels kommen die ortsüblichen Aufwandmengen an den jeweiligen Herbiziden zur Anwendung. Die Aufwandmengen an herbizidem Wirkstoff schwanken zwischen 0,5 und 5 kg/ha. Die Aufwandmenge an Gegenmittel ist unabhängig vom Herbizid und der Aufwandmenge des herbiziden Wirkstoffes. Im allgemeinen liegen die Aufwandmengen an erfindungsgemäßen Gegenmitteln bei der Flächenbehandlung zwischen 0,1 und 5 kg/ha, vorzugsweise zwischen 0,2 und 4 kg/ha. Bei der Saatgutbehandlung liegen die Aufwandmengen an erfindungsgemäßen Gegenmitteln im allgemeinen zwischen 10 und 300 g pro Kilogramm Saatgut, vorzugsweise zwischen 25 und 200 g pro Kilogramm Saatgut.

In den erfindungsgemäßen Wirkstoffkombinationen können die Gewichtsverhältnisse von Gegenmitteln zu herbiziden Wirkstoffen in relativ großen Bereichen

schwanken. Im allgemeinen entfallen auf 1 Gewichtsteil an herbizidem Wirkstoff 0,05 bis 1,0 Gewichtsteile, vorzugsweise 0,1 bis 0,5 Gewichtsteile an Gegenmittel der Formel (I).

Die gute Wirksamkeit der erfindungsgemäßen Gegenmittel geht aus dem nachfolgenden Beispiel hervor.

In diesem Beispiel werden die nachstehend angegebenen Verbindungen als Vergleichskomponenten eingesetzt:

(A) =

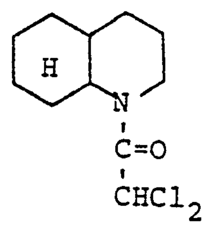

(N-Dichloracetyl-cis,trans-decahydrochinolin)

(B) =

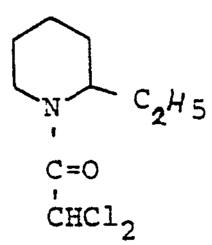

(N-Dichloracetyl-2-ethyl-piperidin)

Ferner wird in diesem Beispiel als herbizider Wirkstoff das nachstehend angegebene Acetanilid eingesetzt:

(C) =

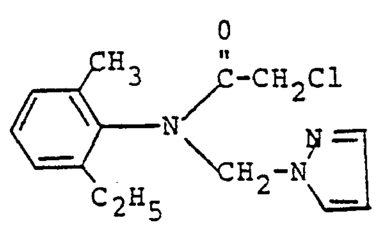

(2-Methyl-6-ethyl-N-(pyrazol-1-yl-methyl)-chloracetanilid)

Beispiel A

Pre-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gew.-Teil herbziden Wirkstoff bzw. Gegenmittel bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 Stunden mit der Herbizid-Zubereitung bzw. mit der Gegenmittel-Zubereitung bzw. mit der Zubereitung aus Gegenmittel und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine Auswertung der Testergebnisse zeigt, daß das erfindungsgemäße N-($\alpha$-Chlorpropionyl)-1,2,3,4-tetrahydro-isochinolins der Formel (I) besser zum Schutz von Kulturpflanzen vor Schäden durch den herbiziden Wirkstoff (C) geeignet ist als die Vergleichskomponenten (A) und (B). Ferner besitzt die Kombination aus (I) und (C) eine bessere Selektivität als die Kombinationen (A) + (C) bzw. (B) + (C).

0023307

Tabelle A

Pre-emergence-Test

| Wirkstoff Herbizid | Wirkstoffaufwand an Herbizid kg/ha | Wirkstoff Gegenmittel | Wirkstoffaufwand an Gegenmittel kg/ha | % Schädigung bei Mais | Echinochloa | Amaranthus |
|---|---|---|---|---|---|---|
| - | - | 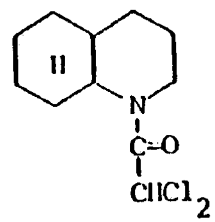 (bekannt) | 3 | 0 | 0 | 0 |
| - | - | 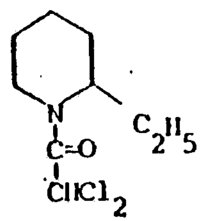 (bekannt) | 3 | 0 | 0 | 0 |
| - | - | 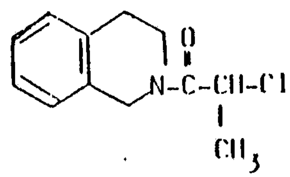 (erfindungsgemäß) | 3 | 0 | 0 | 0 |

Tabelle A (Fortsetzung)

pre-emergence-Test

| Wirkstoff Herbizid | Wirkstoffaufwand an Herbizid kg/ha | Wirkstoff Gegenmittel | Wirkstoffaufwand an Gegenmittel kg/ha | % Schädigung bei Mais | Echinochloa | Amaranthus |
|---|---|---|---|---|---|---|
| 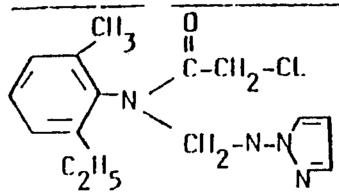 | 3 | - | - | 90 | 100 | 100 |
| 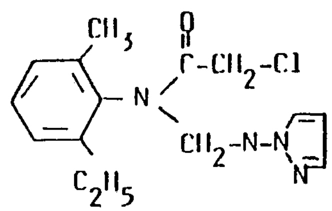 | 3 | 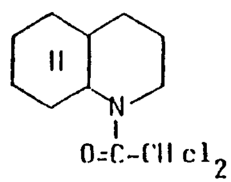 | 3 | 80 | 100 | 100 |
| 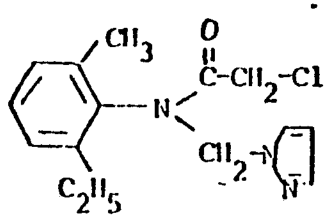 | 3 | 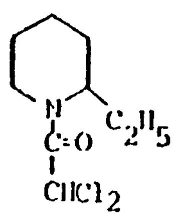 (bekannt) | 3 | 70 | 100 | 100 |
| 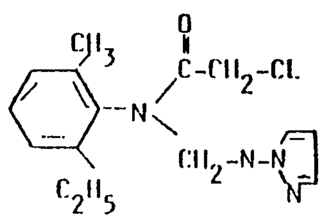 | 3 | 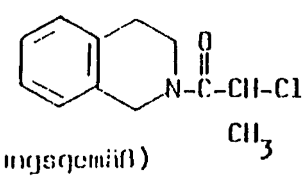 (erfindungsgemäß) | 3 | 30 | 100 | 100 |

## Herstellungsbeispiele

### Beispiel 1

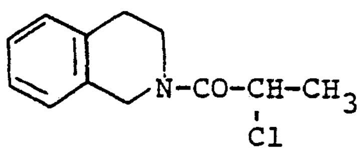

Zu einer Lösung von 26,6 g (0,2 Mol) 1,2,3,4-Tetrahydroisochinolin in 150 ml Acetonitril werden 12,7 g (0,1 Mol) 2-Chlorpropionsäurechlorid in der Art getropft, daß die Reaktionstemperatur 40°C nicht übersteigt.

Es wird 2 Stunden bei 40°C nachgerührt und dann abgekühlt. Zum Reaktionsgemisch werden 150 ml Wasser und 200 ml Toluol gegeben. Die Toluollösung wird abgetrennt, über Natriumsulfat getrocknet und eingeengt. Der Rückstand wird andestilliert.

Man erhält auf diese Weise 12 g (54 % der Theorie) an N-($\alpha$-Chlorpropionyl)-1,2,3,4-tetrahydro-isochinolin in Form eines hellgelben Öls vom Brechungsindex $n_D^{22}$ = 1,5701.

### Beispiel 2

Herstellung des Ausgangsproduktes:

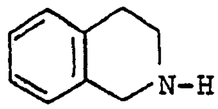

129 g (1 Mol) Isochinolin werden mit 15 g eines Katalysatorgemisches, bestehend aus Ruthenium auf Aluminiumoxid, versetzt und 2 Stunden lang bei 190°C unter einem Wasserstoffdruck von 170 bis 190 bar hydriert. Danach wird das Reaktionsgemisch filtriert und einer fraktionierten Destillation unterworfen. Man erhält auf diese Weise 115 g an 1,2,3,4-Tetrahydro-isochinolin.
Siedepunkt: 115°C bei 24 mm Hg.

Beispiel (V-1)

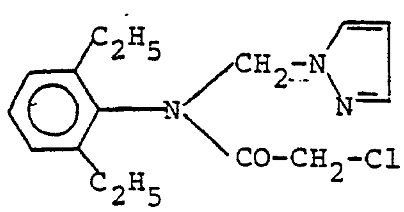

Zu 274,2 g (1 Mol) 2,6-Diethyl-N-chlormethylchloracetanilid in 250 ml wasserfreiem Essigester gibt man unter Rühren eine Mischung aus 68 g (1 Mol) Pyrazol und 106 g (1,05 Mol) Triethylamin in 150 ml wasserfreiem Essigester, wobei die Temperatur auf 30°C ansteigt. Man rührt 1 Stunde bei Raumtemperatur nach. Für die Aufarbeitung ergeben sich zwei Möglichkeiten:

1) Das Reaktionsgemisch wird filtriert, das Filtrat mit Wasser neutral gewaschen, über Natriumsulfat getrocknet und im Vakuum eingedampft. Nach eine fraktionierten Kristallisation mit Ligroin erhält man 171,2 g (56 % der Theorie) 2,6-Diethyl-N-(pyrazol-1-yl-methyl)-chloracetanilid vom Schmelzpunkt 67°C in Form farbloser Kristalle.

2) Das Reaktionsgemisch wird auf 0°C abgekühlt, filtriert und der Filterrückstand mit 10 ml kaltem Essigester nachgewaschen. In das Filtrat werden bei 0 bis -10°C 50 g (1,4 Mol) trockener Chlorwasserstoff eingeleitet. Man saugt anschließend die ausgefallenen Hydrochlorid-Salze ab, wäscht mit 50 ml kaltem Essigester nach und verteilt den festen Rückstand zwischen 0,5 l Essigester und 0,5 l wäßriger Natriumhydroxid-Lösung mit einem pH-Wert von 12. Die organische Phase wird abgetrennt, zweimal mit je 0,5 l Natriumchloridlösung gewaschen, über Natriumsulfat getrocknet und im Vakuum eingedampft. Der farblose ölige Rückstand wird mit 60 ml Benzin versetzt, wobei er kristallisiert. Man erhält 220,2 g (72 % der Theorie) 2,6-Diethyl-N-(pyrazol-1-yl-methyl)-chloracetanilid vom Schmelzpunkt 67°C in Form farbloser Kristalle.

In analoger Weise werden die in der nachfolgenden Tabelle aufgeführten Verbindungen hergestellt:

Tabelle 1

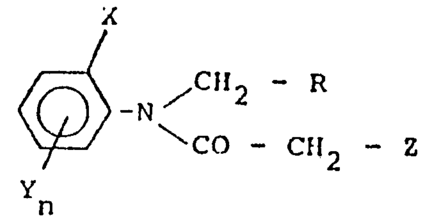

(V)

| Bsp.-Nr. | X | Yn | Z | R | Schmelzpunkt (°C) |
|---|---|---|---|---|---|
| V-2 | $C_2H_5$ | 6-$C_2H_5$ | Cl | 1,2,4-Triazol-1-yl | 112 |
| V-3 | i-$C_3H_7$ | 6-i-$C_3H_7$ | Cl | Pyrazol-1-yl | 134 |
| V-4 | $CH_3$ | 6-$C_2H_5$ | Cl | 1,2,4-Triazol-1-yl | 92 |
| V-5 | $CH_3$ | 6-$C_2H_5$ | Cl | Pyrazol-1-yl | 57 |
| V-6 | $C_2H_5$ | 4,6-$(CH_3)_2$ | Cl | Pyrazol-1-yl | 32 |
| V-7 | $CH_3$ | 4,6-$(CH_3)_2$ | Cl | Pyrazol-1-yl | 92 |
| V-8 | $C_2H_5$ | 4-$CH_3$, 6-$C_2H_5$ | Cl | Pyrazol-1-yl | 78 |
| V-9 | i-$C_3H_7$ | 6-i-$C_3H_7$ | Cl | 1,3,4-Triazol-1-yl | 196 |
| V-10 | i-$C_3H_7$ | 6-i-$C_3H_7$ | Cl | 1,2,4-Triazol-1-yl | 138 |
| V-11 | $C_2H_5$ | 6-$C_2H_5$ | Cl | Pyrrol-1-yl | Oel |
| V-12 | i-$C_3H_7$ | - | Cl | 1,2,4-Triazol-1-yl | 118 |
| V-13 | $CH_3$ | 6-$C_2H_5$ | Cl | 1,2,3,4-Tetrazol-1-yl | Oel |
| V-14 | i-$C_3H_7$ | - | Cl | Pyrazol-1-yl | Oel |

Tabelle 1 (Mortsetzung)

| Bsp.-Nr. | X | Yn | Z | R | Schmelzpunkt (°C) |
|---|---|---|---|---|---|
| V-15 | $C_2H_5$ | - | Cl | 1,2,4-Triazol-1-yl | 81 |
| V-16 | $CH_3$ | $6-CH_3$ | Cl | Pyrazol-1-yl | 82 |
| V-17 | $CH_3$ | $6-CH_3$ | Cl | 1,2,4-Triazol-1-yl | 110 |
| V-18 | $CH_3$ | $5-CH_3$ | Cl | 1,2,4-Triazol-1-yl | Oel |
| V-19 | $CH_3$ | - | Cl | Pyrazol-1-yl | 56 |
| V-20 | $CH_3$ | - | Cl | 1,2,4-Triazol-1-yl | 88 |
| V-21 | $CH_3$ | $5-CH_3$ | Cl | Pyrazol-1-yl | Oel |
| V-22 | $CH_2$ | $3-CH_3$ | Cl | 1,2,4-Triazol-1-yl | 114 |
| V-23 | $CH_3$ | $3-CH_3$ | Cl | Pyrazol-1-yl | 102 |
| V-24 | $C_2H_5$ | $6-CH_3$ | Cl | Pyrazol-1-yl (xHCl) | 87 |
| V-25 | $C_2H_5$ | $6-C_2H_5$ | Cl | Pyrazol-1-yl (xHCl) | 67 |
| V-26 | $C_2H_5$ | $6-C_2H_5$ | Cl | 3,5-Dimethyl-pyrazol-1-yl | 111 |
| V-27 | $C_2H_3$ | $6-C_2H_3$ | Cl | Brom-methyl-pyrazolyl | 145 |
| V-28 | $C_2H_5$ | $6-C_2H_5$ | Cl | 3-Chlor-1,2,4-triazol-1-yl | 110 |
| V-29 | $CH_3$ | $6-C_2H_5$ | Cl | 3,5-Dimethyl-pyrazol-1-yl | 90 |

Tabelle 1 (Fortsetzung)

| Bsp.-Nr. | X | Yn | Z | R | Schmelzpunkt (°C) |
|---|---|---|---|---|---|
| V-30 | $C_2H_3$ | 6-$C_2H_3$ | Cl | 3-Methyl-pyrazol-1-yl | 89 |
| V-31 | $C_2H_5$ | 6-$CH_3$ | Cl | 3-Methyl-pyrazol-1-yl | 113 |
| V-32 | $C(CH_3)_3$ | - | Cl | Pyrazol-1-yl | Oel |
| V-33 | $C(CH_3)_2$ | - | Cl | 1,2,4-Triazol-1-yl | 118 |
| V-34 | $C_2H_5$ | 6-$CH_3$ | Cl | Brom-methyl-pyrazolyl | 80 |
| V-35 | $CH_3$ | 6-$C_2H_5$ | Cl | 4-Chlor-pyrazol-1-yl | 91 |
| V-36 | $CH_3$ | 6-$C_2H_5$ | Cl | 3-Chlor-1,2,4-triazol-1-yl | 121 |
| V-37 | $C_2H_5$ | 6-$CH_3$ | Cl | 2,4,5-Trichlorimidazol-1-yl | 158 |
| V-38 | $C_2H_5$ | 6-$C_2H_5$ | Cl | 4-Chlor-pyrazol-1-yl | 110 |
| V-39 | $C_2H_5$ | 6-$C_2H_5$ | Cl | 1,2,3,4-Tetrazol-1-yl | 110 |
| V-40 | $C_2H_5$ | 6-$C_2H_5$ | Br | Pyrazol-1-yl | 68 |
| V-41 | $CH_3$ | 6-$C_2H_5$ | Br | Pyrazol-1-yl | 67 |
| V-42 | $C_2H_5$ | 6-$C_2H_5$ | Cl | Imidazol-1-yl | Oel |
| V-43 | $C_2H_5$ | 6-$C_2H_5$ | Br | 1,2,4-Triazol-1-yl | 90 |
| V-44 | $CH_3$ | 6-$C_2H_5$ | Br | 1,2,4-Triazol-1-yl | 78 |

Beispiel (VI-1)

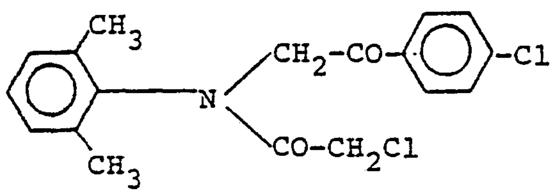

In eine Lösung von 18,5 g (0,068 Mol) 2,6-Dimethyl-N-(4-chlor-benzoylmethyl)-anilin in 150 ml Benzol werden 16 ml (0,2 Mol) Chloracetylchlorid getropft. Danach läßt man 15 Stunden unter Rückfluß rühren und engt durch Abdestillieren des Lösungsmittels und des überschüssigen Chloracetylchlorids im Vakuum ein. Der Rückstand wird mit einem Gemisch Ether/Petrolether (1:3) verrieben, der entstehende kristalline Rückstand abgesaugt und getrocknet. Man erhält 17,7 g (75 % der Theorie) 2,6-Dimethyl-N-(4-chlorbenzoylmethyl)-chloracetanilid vom Schmelzpunkt 128°C.

Beispiel (VI-2)

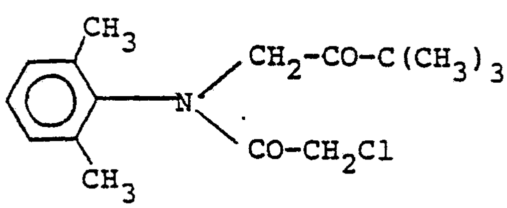

23,3 g (0,1 Mol) 2-Ethyl-6-methyl-N-pivaloylmethylanilin werden in 100 ml Benzol gelöst und mit 24 ml (0,3 Mol) Chloracetylchlorid versetzt. Danach läßt

man 15 Stunden unter Rückfluß rühren und engt durch Abdestillieren des Lösungsmittels und des überschüssigen Chloracetylchlorids im Vakuum ein. Der ölige Rückstand wird mit Petrolether verrührt, dekantiert, mit Aktivkohle verrührt, filtriert und im Vakuum eingeengt. Der Rückstand wird mit n-Hexan verrührt, der resultierende Feststoff abgesaugt und getrocknet. Man erhält 13,7 g (45 % der Theorie) 2-Ethyl-6-methyl-N-pivaloylmethyl-chloracetanilid vom Schmelzpunkt 86°C.

Nach der in den Beispielen (VI-1) und (VI-2) beschriebenen Methode werden auch die in der nachfolgenden Tabelle 2 aufgeführten Verbindungen hergestellt:

Tabelle 2

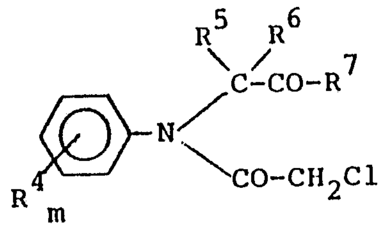

(VI)

| Bsp.-Nr. | $R^4_m$ | $R^5$ | $R^6$ | $R^7$ | Schmelzpunkt (°C) bzw. Brechungsindex |
|---|---|---|---|---|---|
| VI-3 | 2-$CH_3$ | H | H | 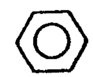 | 138 |
| VI-4 | 2-$CH_3$ | H | H | 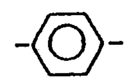 | 140 |
| VI-5 | 2,6-$(C_2H_5)_2$ | H | H | 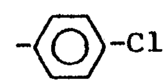 | 134 |
| VI-6 | 2,6-$(C_2H_5)_2$ | H | H | 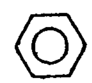 | 116 |
| VI-7 | 2-Cl | H | H | 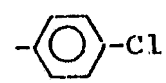 | 124 |
| VI-8 | 2,6-$(CH_3)_2$ | H | H | 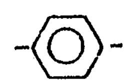 | 100 |
| VI-9 | 4-Cl | H | H | 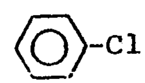 | 114 |
| VI-10 | 2,6-$(CH_3)_2$ | $CH_3$ | H | $CH_3$ | 104 |

Tabelle 2 (Fortsetzung)

| Bsp.-Nr. | $R^4_m$ | $R^5$ | $R^6$ | $R^7$ | Schmelzpunkt (°C) bzw. Brechungsindex |
|---|---|---|---|---|---|
| VI-11 | 2,6-(i-$C_3H_7$)$_2$ | H | H | 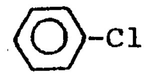 | 200 |
| VI-12 | 2,6-($C_2H_5$)$_2$, 4-$CH_3$ | H | H | 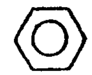 | 112 |
| VI-13 | 2,6-(i-$C_3H_7$)$_2$ | H | H | 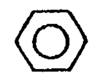 | 140 |
| VI-14 | 2,6-($CH_3$)$_2$ | H | H | 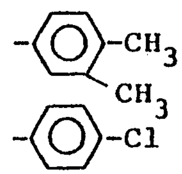 | 90 |
| VI-15 | 2-$C_2H_5$, 6-$CH_3$ | H | H | | 70 |
| VI-16 | 2,6-($CH_3$)$_2$ | H | H | 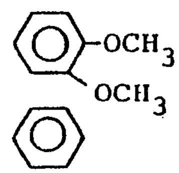 | 114 |
| VI-17 | 2-$C_2H_5$, 4,6-($CH_3$)$_2$ | H | H | | $n_D^{20}$ = 1,5680 |
| VI-18 | 2,6-($CH_3$)$_2$ | H | H | 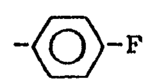 | 104 |
| VI-19 | 2,4,6-($CH_3$) | H | H | 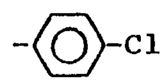 | 134 |

Tabelle 2 (Fortsetzung)

| Bsp.-Nr. | $R^4_m$ | $R^5$ | $R^6$ | $R^7$ | Schmelzpunkt (°C) bzw. Brechungsindex |
|---|---|---|---|---|---|
| VI-20 | 2,4,6-$(CH_3)_3$ | H | H | 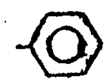 | $n_D^{20}$ = 1,5610 |
| VI-21 | 2,6-$(CH_3)_2$ | H | 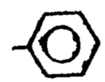 | 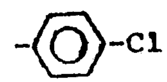 | 149 |
| VI-22 | 2,6-$(CH_3)_2$ | H | $CH_3$ | 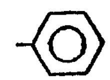 | 84 |



BAD ORIGINAL

In analoger Weise können die in der nachfolgenden Tabelle 3 aufgeführten Verbindungen erhalten werden.

Tabelle 3

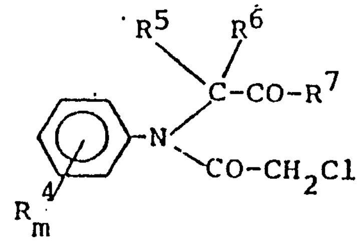

(VI)

| Bsp.-Nr. | $R^4_m$ | $R^5$ | $R^6$ | $R^7$ |
|---|---|---|---|---|
| VI-23 | 3,5-$(CF_3)_2$ | H | H | 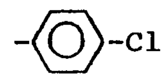 |
| VI-24 | 2,6-$(CH_3)_2$ | H | H | 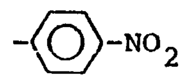 |
| VI-25 | 2,6-$(CH_3)_2$ | H | H | 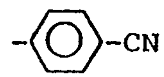 |
| VI-26 | 2,6-$(CH_3)_2$ | H | H | 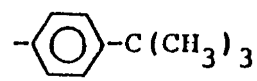 |
| VI-27 | 2,6-$(CH_3)_2$, 4-$SO_2NH_2$ | H | H | 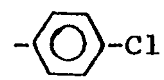 |
| VI-28 | 2-Cl, 6-$CH_3$ | H | H | 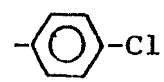 |

Tabelle 3 (Fortsetzung)

| Bsp.-Nr. | $R^4_m$ | $R^5$ | $R^6$ | $R^7$ |
|---|---|---|---|---|
| VI-29 | $2-C_2H_5$, $6-CH_3$ | $CH_3$ | $CH_3$ | 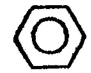 |
| VI-30 | $2-C_2H_5$, $6-CH_3$ | $CH_3$ | $CH_3$ | 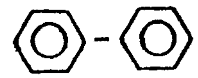 |
| VI-31 | $2-C_2H_5$, $6-CH_3$ | $CH_3$ | $CH_3$ | 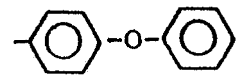 |
| VI-32 | $2-C_2H_5$, $6-CH_3$ | $CH_3$ | $CH_3$ | 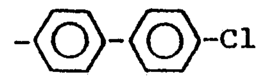 |
| VI-33 | $2-C_3H_5$, $6-CH_3$ | $CH_3$ | $CH_3$ | 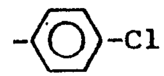 |
| VI-34 | $2-C_2H_5$, $6-CH_3$ | H | $CH_3$ | 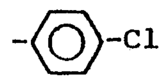 |
| VI-35 | $2-C_2H_5$, $6-CH_3$ | H | $CH_3$ | 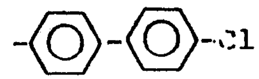 |
| VI-36 | $2,6-(CH_3)_2$ | H | 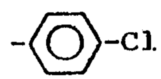 | 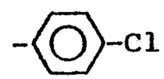 |
| VI-37 | $2,6-(CH_3)_2$ | H | 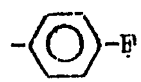 | 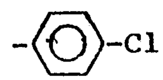 |

Tabelle 3 (Fortsetzung)

| Bsp.-Nr. | $R^4_m$ | $R^5$ | $R^6$ | $R^7$ |
|---|---|---|---|---|
| VI-38 | 2,6-$(CH_3)_2$ | H | 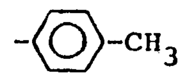 | 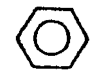 |
| VI-39 | 2,6-$(CH_3)_2$ | H | 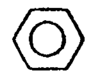 | 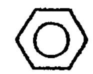 |
| VI-40 | 2,6-$(CH_3)_2$ | H | 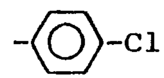 | 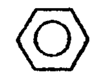 |
| VI-41 | 2,6-$(CH_3)_2$ | H | $CH_3$ | 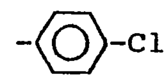 |

Beispiel (VII-1)

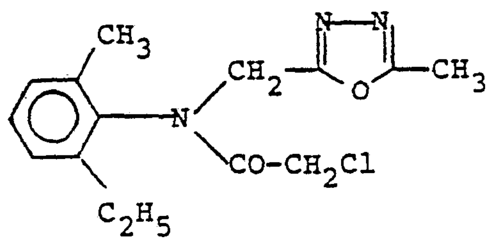

16,3 g (0,07 Mol) 2-Ethyl-6-methyl-N-[(2-methyl-1,3,4-oxadiazol-5-yl)-methyl]-anilin und 6 g (0,076 Mol) wasserfreies Pyridin werden in 100 ml absolutem Tetrahydrofuran unter Rühren zum Sieden erhitzt und tropfenweise mit einer Lösung von 8 g (0,07 Mol) Chloracetylchlorid in 20 ml Tetrahydrofuran versetzt. Nach beendetem Zutropfen läßt man 10 Minuten nachrühren, engt durch Abdestillieren des Lösungsmittels ein und verrührt den Rückstand mit 150 ml Wasser. Das auskristallisierende Reaktionsprodukt wird abgesaugt, mit Wasser gewaschen und getrocknet. Man erhält 18,7 g (87 % der Theorie) beige-farbene Kristalle von 2-Ethyl-6-methyl-N-[(2-methyl-1,3,4-oxadiazol-5-yl)-methyl]-chloracetanilid vom Schmelzpunkt 67 bis 70°C.

Herstellung des Ausgangsproduktes

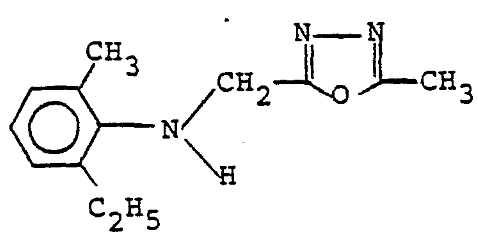

Eine Mischung aus 101,2 g (0,67 Mol) 2-Ethyl-6-methyl-anilin, 40 g (0,3 Mol) 2-Methyl-5-chlormethyl-1,3,4-oxadiazol, 41,1 g (0,3 Mol) gepulvertes Kaliumcarbonat und 76 ml Dimethylformamid wird 5 Stunden unter Rühren auf 100°C erhitzt. Danach wird die Reaktionsmischung filtriert, das Filtrat mit Methylenchlorid verdünnt und mehrmals mit Wasser gewaschen. Die Methylenchloridphase wird über Natriumsulfat getrocknet und im Vakuum durch Abdestillieren des Lösungsmittels eingeengt. Der Rückstand wird im Vakuum destilliert. Man erhält 46,8 g (67,5 % der Theorie) gelbliches Öl von 2-Ethyl-6-methyl-N-[(2-methyl-1,3,4-oxadiazol-5-yl)-methyl]-anilin vom Siedepunkt 140 bis 142°C/0,1 mm mit einer 94 %igen Reinheit (gaschromatographisch bestimmt).

Beispiel (VII-2)

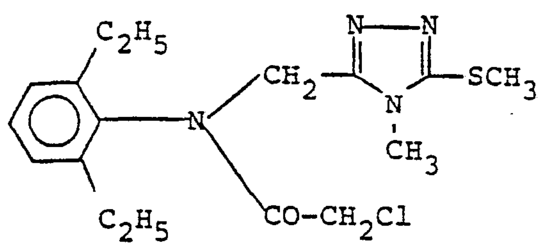

5 g (0,017 Mol) 2,6-Diethyl-N-[(1-methyl-2-methylthio-1,3,4-triazol-5-yl)-methyl]-anilin und 1,6 g (0,02 Mol) Pyridin werden in 100 ml absolutem Tetrahydrofuran gerührt und bei Raumtemperatur tropfenweise mit 2,3 g (0,02 Mol) Chloracetylchlorid

versetzt, wobei die Temperatur auf ca. 30°C ansteigt. Man läßt 2 Stunden rühren, engt teilweise durch Abdestillieren des Lösungsmittels ein und versetzt mit Wasser. Das auskristallisierende Produkt wird abgesaugt, getrocknet und aus Diisopropylether/Essigester umkristallisiert. Man erhält 5 g (80 % der Theorie) 2,6-Diethyl-N-[(1-methyl-2-methylthio-1,3,4-triazol-5-yl)-methyl]-chloracetanilid vom Schmelzpunkt 121 bis 123°C.

Herstellung der Vorstufen

a)

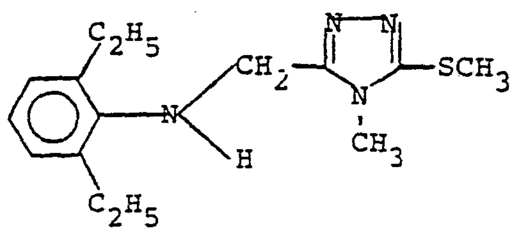

13,9 g (0,05 Mol) 2,6-Diethyl-N-[(1-methyl-2-thiono-1,3,4-triazol-5-yl)-methyl]-anilin werden bei Raumtemperatur in einem Zweiphasengemisch aus 150 ml Toluol und 40 ml 50 %iger Natronlauge unter Zusatz von 1,5 g Triethyl-benzyl-ammoniumchlorid (TEBA) als Katalysator schnell gerührt und tropfenweise mit 6,3 g (0,05 Mol) Dimethylsulfat versetzt, wobei die Temperatur auf ca. 35°C ansteigt. Man läßt 5 Stunden rühren, trennt die Toluolphase ab, wäscht sie mehrmals mit Wasser, trocknet über Natriumsulfat und engt durch Abdestillieren des Lösungsmittels ein. Das zurückbleibende Öl wird

durch Zusatz von Petrolether zur Kristallisation gebracht. Man erhält nach Umkristallisation aus Petrolether 6,7 g (40 % der Theorie) 2,6-Diethyl-N-[(1-methyl-2-methylthio-1,3,4-triazol-5-yl)-methyl]-anilin vom Schmelzpunkt 65 bis 67°C.

b) 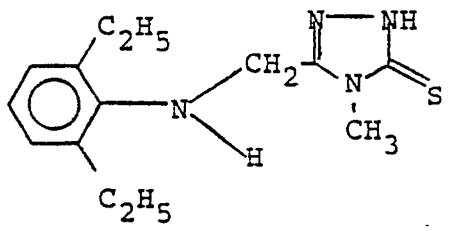

29,6 g (0,1 Mol) 1-Methyl-4-[(2,6-diethyl-anilino)-acetyl]-thiosemicarbazid werden in 150 ml Ethanol suspendiert und nach Zugabe von 7 g Kaliumhydroxid in 20 ml Wasser 1 Stunde unter Rückfluß erhitzt. Danach wird der größte Teil des Lösungsmittels abdestilliert und der Rückstand mit 250 ml Wasser versetzt. Nach dem Ansäuern mit Eisessig auf pH 5 wird der entstehende Niederschlag abgesaugt und gründlich mit Wasser gewaschen. Nach dem Trocknen erhält man man 27 g (97 % der Theorie) 2,6-Diethyl-N-[(1-methyl-2-thiono-1,3,4-triazol-5-yl)-methyl]-anilin vom Schmelzpunkt 117 bis 121°C.

c) 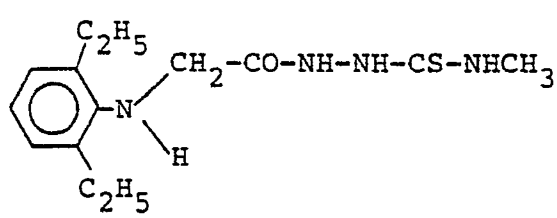

44,2 g (0,2 Mol) 2,6-Diethyl-anilino-essigsäurehydrazid und 14,8 g (0,2 Mol) Methylsenföl werden in 250 ml Ethanol gelöst und eine Stunde auf Rückflußtemperatur erhitzt. Nach dem anschließenden Abkühlen auf Raumtemperatur wird der entstandene Niederschlag abgesaugt und zweimal mit je 50 ml Ethanol nachgewaschen. Nach dem Trocknen erhält man 46 g (78 % der Theorie) an 1-Methyl-4-[(2,6-diethyl-anilino)-acetyl]-thiosemicarbazid in Form einer farblosen kristallinen Substanz vom Schmelzpunkt 166°C.

d) 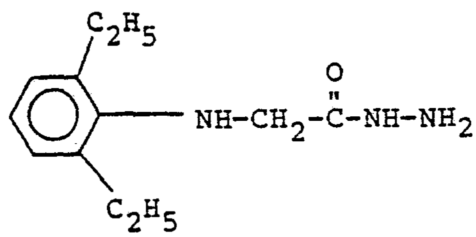

58,7 g (0,25 Mol) 2,6-Diethyl-anilino-essigsäureethyl-ester und 25 g Hydrazinhydrat werden in 200 ml Ethanol 24 Stunden stehen gelassen. Danach wird durch Abdestillieren des Lösungsmittels eingeengt und der Rückstand mit Wasser ausgerührt. Nach dem Trocknen erhält man 50,5 g (91 % der Theorie) farblose Kristalle von 2,6-Diethyl-anilino-essigsäurehydrazid vom Schmelzpunkt 71 bis 73°C.

In entsprechender Weise werden diejenigen Verbindungen erhalten, die in der Tabelle 4 formelmäßig aufgeführt sind.

Tabelle 4

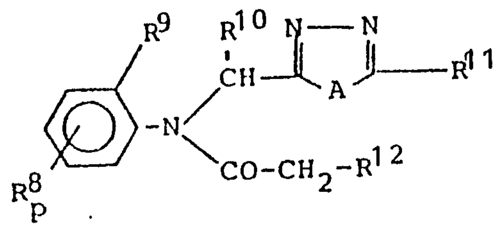

(VII)

| Bsp.-Nr. | $R^{10}$ | $R^{11}$ | $R^9$ | $R^8_p$ | A | $R^{12}$ | Schmelzpunkt (°C) |
|---|---|---|---|---|---|---|---|
| VII-3 | H | $CH_3$ | $C_2H_5$ | $6-C_2H_5$ | O | Cl | 97 - 82 |
| VII-4 | H | $CH_3$ | $CH_3$ | $6-CH_3$ | O | Cl | 91 - 93 |
| VII-5 | H | $CH_3$ | $C(CH_3)_3$ | - | O | Cl | 102 - 04 |
| VII-6 | H | $-S-CH_2-CH=CH_2$ | $C_2H_5$ | $6-C_2H_5$ | 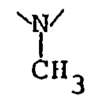 | Cl | 67 - 70° |
| VII-7 | H | $-S-CH_2-$ 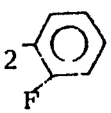 | $CH_3$ | $6-C_3H_5$ | 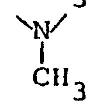 | Cl | 115 - 20 |
| VII-8 | H | $C_2H_5$ | $CH_3$ | $6-C_2H_5$ | O | Cl | 57 - 59 |
| VII-9 | H | $C_2H_5$ | $C_2H_5$ | $6-C_2H_5$ | O | Cl | 43 - 47 |
| VII-10 | H | $i-C_3H_7$ | $CH_3$ | $6-C_2H_5$ | O | Cl | zähfl. Oel |
| VII-11 | H | $CH_3$ | $CH_3$ | $3-CH_3$ | 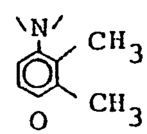 | Cl | glasartig erstarrt |
| VII-12 | H | $CH_3$ | $C_2H_5$ | $6-C_2H_5$ | O | Br | 80° |
| VII-13 | H | $CH_3$ | $CH_3$ | $6-C_2H_5$ | O | Br | 92 - 94°C |
| VII-14 | H | $CH_3$ | $i-C_3H_7$ | $6-i-C_3H_7$ | O | Cl | 135 - 37°C |

Nach einem oder mehreren der in der Anmeldung beschriebenen Verfahren werden die in der nachstehenden Tabelle formelmäßig aufgeführten Ausgangsprodukte erhalten.

Tabelle 5

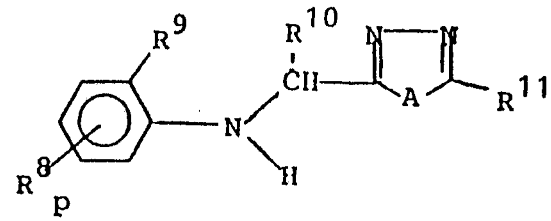

(X)

| Bsp.-Nr. | $R^{10}$ | $R^{11}$ | $R^9$ | $R^8$ | A | Schmelzpunkt (°C) bzw. Brechungsindex |
|---|---|---|---|---|---|---|
| X-1 | H | $CH_3$ | $C_2H_5$ | $6-C_2H_5$ | 0 | $n_D^{22} = 1,540$ |
| X-2 | H | $CH_3$ | $CH_3$ | $6-C_2H_5$ | 0 | $n_D^{22} = 1,347$ |
| X-3 | H | $CH_3$ | $CH_3$ | $6-CH_3$ | 0 | $n_D^{22} = 1,552$ |
| X-4 | H | $CH_3$ | $-(CH_3)_3$ | - | 0 | 52 - 55 |
| X-5 | H | $CH_3$ | $i-C_3H_7$ | $6-i-C_3H_7$ | 0 | 96 - 99 |
| X-6 | H | $C_2H_5$ | $C_2H_5$ | $6-C_2H_5$ | 0 | $n_D^{22} = 1,534$ |
| X-7 | H | $C_2H_5$ | $CH_3$ | $6-C_2H_5$ | 0 | $n_D^{21} = 1,342$ |
| X-8 | H | $i-C_3H_7$ | $CH_3$ | $6-C_2H_5$ | 0 | $n_D^{21} = 1,531$ |
| X-9 | H | $SCH_3$ | $C_2H_5$ | $6-C_2H_5$ | $N-CH_3$ | 65 - 57 |
| X-10 | H | $S-CH_2-CH=CH_2$ | $C_2H_5$ | $6-C_2H_5$ | $N-CH_3$ | $n_D^{21} = 1,577$ |

Tabelle 5 (Fortsetzung)

| Bsp.-Nr. | $R^{10}$ | $R^{11}$ | $R^9$ | $R^8_p$ | A | Schmelzpunkt [°C] bzw. Brechungsindex |
|---|---|---|---|---|---|---|
| X-11 | H | $S-CH_2-$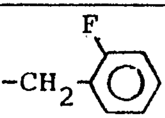 | $CH_3$ | $6-C_2H_5$ | $N-CH_3$ | zähes Öl |
| X-12 | H | $CH_3$ | $CH_3$ | $3-CH_3$ | 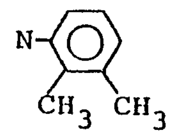 | 142 - 143 |

Patentansprüche

1. N-(α-Chlorpropionyl)-1,2,3,4-tetrahydro-isochinolin der Formel

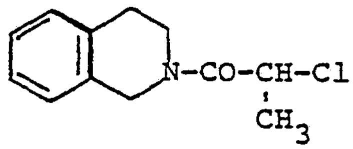

(I)

2. Verfahren zur Herstellung von N-(α-Chlor-propionyl)-1,2,3,4-tetrahydro-isochinolin der Formel (I), dadurch gekennzeichnet, daß man 1,2,3,4-Tetrahydro-isochinolin der Formel

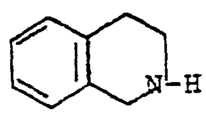

(II)

mit α-Chlor-propionsäurechlorid der Formel

$$Cl-CO-\underset{Cl}{CH}-CH_3$$ (III)

gegebenenfalls in Gegenwart eines Säurebindemittels sowie gegebenenfalls in Gegenwart eines Verdünnungsmittels umsetzt.

3. Mittel zum Schutz von Kulturpflanzen vor Schädigungen durch herbizid wirksame Thiolcarbamate bzw. durch herbizid wirksame Acetanilide, gekennzeichnet durch einen Gehalt an N-(α-

Chlorpropionyl)-1,2,3,4-tetrahydro-isochinolin der Formel (I) gemäß Anspruch 1.

4. Verfahren zum Schutz von Kulturpflanzen vor Schädigungen durch herbizid wirksame Thiolcarbamate bzw. herbizid wirksame Acetanilide, dadurch gekennzeichnet, daß man N-(α-Chlorpropionyl)-1,2,3,4-tetrahydro-isochinolin der Formel (I) gemäß Anspruch 1 auf die Kulturpflanzen und/oder deren Lebensraum einwirken läßt.

5. Verwendung von N-(α-Chlorpropionyl)-1,2,3,4-tetrahydro-isochinolin der Formel (I) gemäß Anspruch 1 zum Schutz von Kulturpflanzen vor Schädigungen durch herbizid wirksame Thiolcarbamate bzw. durch herbizid wirksame Acetanilide.

6. Verfahren zur Herstellung von Mitteln zum Schutz von Kulturpflanzen vor Schädigungen durch herbizid wirksame Thiolcarbamate bzw. herbizid wirksame Acetanilide, dadurch gekennzeichnet, daß man N-(α-Chlorpropionyl)-1,2,3,4-tetrahydro-isochinolin der Formel (I) gemäß Anspruch 1 mit Streckmitteln und/oder oberflächenaktiven Stoffen vermischt.

7. Mittel zur selektiven Unkrautbekämpfung in Nutzpflanzenkulturen, gekennzeichnet durch

einen Gehalt an einer Wirkstoffkombination bestehend aus N-(α-Chlorpropionyl)-1,2,3,4-tetrahydro-isochinolin der Formel (I) gemäß Anspruch 1 und mindestens einem herbizid wirksamen Thiolcarbamat bzw. mindestens einem herbizid wirksamen Acetanilid.

8. Verwendung von Wirkstoffkombinationen gemäß Anspruch 7 zur selektiven Unkrautbekämpfung in Nutzpflanzenkulturen.

0023307

Europäisches Patentamt

# EUROPÄISCHER RECHERCHENBERICHT

Nummer der Anmeldung
EP 80 10 40c3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 218 097 (STAUFFER)<br>* Ansprüche *<br>-- | 1,3 |
| P | EP - A - 0 006 542 (BAYER)<br>* Ansprüche *<br>---- | 1,3 |

KLASSIFIKATION DER ANMELDUNG (Int Cl )

C 07 D 217/06
A 01 N 25/32//
C 07 D 217/04
231/12
231/16
249/08
249/10
249/12
207/32
257/04
233/61
233/68
271/10 ./.

RECHERCHIERTE SACHGEBIETE (Int. Cl )

C 07 D 217/06
A 01 N 25/32

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 30-10-1980 | ALFARO |

0023307

Europäisches Patentamt

# EUROPÄISCHER RECHERCHENBERICHT

Nummer der Anmeldung
EP 80 10 4063
-2-

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | | | C 07 C 103/42<br>109/097<br>159/00 |
| | | | RECHERCHIERTE SACHGEBIETE (Int. Cl.³) |
| | | | |

EPA Form 1503.2 06.78